# EUROPEAN PATENT APPLICATION

(11) **EP 2 578 247 A2**
(43) Date of publication of application: **10.04.2013**
(21) Application number: 12185560.5
(22) Date of filing: 21.09.2012
(51) Int. Cl.: A61L 27/36

(54) **Processing tissue utilizing supercritical fluid**

(30) Priority: 22.09.2011 US 201161537749 P; 19.07.2012 US 201213552701
(71) Applicant: Covidien LP, Mansfield, MA 02048 (US)
(72) Inventor: Gleiman, Seth, Guilford, CT Connecticut 06437 (US); Gould, Jessica, Bethany, CT Connecticut 06524 (US); Aminuddin, Norman, Madison, CT Connecticut 06443 (US)
(74) Representative: Maschio, Antonio

(57) **Abstract**

A method for processing animal-derived tissue and cross-linking animal-derived tissue is disclosed. Each method includes exposing or contacting the animal-derived tissue with a supercritical fluid.

## Description

### CROSS-REFERENCE TO RELATED APPLICATIONS

This application claims the benefit of and priority to U.S. Provisional Patent Application No. 61/537,749, filed September 22, 2011, the entire disclosure of which is incorporated by reference herein.

### BACKGROUND

The present disclosure relates to medical devices which include animal-derived tissue and methods for preparing the thereof.

Various types of implants are commonly used in biomedical applications. Implants are used in repair of hard tissue (e.g., cartilage, bone, etc.) as well as soft tissue (e.g., muscle, connective tissue, etc.). Medical devices may comprise, in whole or in part, animal-derived tissue such as collagen. Methods for preparing and processing animal-derived tissue remain time consuming and costly. Improvements in the field are desired.

### SUMMARY

Disclosed herein is a method for processing animal-derived tissue. In other embodiments, a method for cross-linking animal-derived tissue is disclosed. These methods may include the use of supercritical fluids such as supercritical nitrogen dioxide and/or supercritical carbon dioxide.

### DETAILED DESCRIPTION

The present disclosure provides a method for cross-linking and processing material, including animal-derived tissue via supercritical fluid. As used herein, the term "supercritical fluid" may be used interchangeably with "densified fluid" and refers to any composition that is above a temperature and pressure at which the phase boundary (e.g., between liquid, gas, or solid) does not exist, i.e., critical state.

Animal-derived tissues included herein may comprise poly(amino acids) including proteins such as collagen (I, II and III), elastin, fibrin, fibrinogen, and albumin; peptides including sequences for laminin and fibronectin (RGD); polysaccharides such as hyaluronic acid (HA); glycosaminoglycan; gut; and combinations thereof. Additionally, as used herein, collagen includes natural collagen such as animal-derived collagen, gelatinized collagen, and/or synthetic collagen such as human or bacterial recombinant collagen.

In specific embodiments, the animal-derived tissue may be composed of porcine dermal collagen. The collagen is an acellular porcine dermal collagen, free of cell, cell debris, RNA and DNA.

The animal-derived tissue may be of any form and structure including, but not limited to, meshes, foams, fibers, grafts, films, particles, and the like. Where the animal-derived tissue is fibrous, the tissue may be formed using any method suitable for forming fibrous structures including, but not limited, to knitting, weaving, non-woven techniques, wet-spinning, electro-spinning, extrusion, co-extrusion, and the like. In embodiments, the animal-derived tissue may be a textile having a three-dimensional structure, such as the textiles described in U.S. Patent Nos. 7,021,086 and 6,443,964, the entire disclosures of each of which are incorporated by reference herein.

In embodiments where the animal-derived tissue is a foam, the tissue may be formed using any method suitable to forming a foam or sponge including, but not limited to, lyophilization or freeze-drying of a composition. In embodiments, a porous substrate may be formed by foaming supercritical carbon dioxide through tissue as described in H. Tai et al., "Putting The Fizz Into Chemistry: Applications of Supercritical Carbon Dioxide in Tissue Engineering, Drug Delivery and Synthesis of Novel Block Copolymers," Biochem. Soc. Trans. 35 (2007), pp. 516 - 521, the entire disclosure of which is incorporated by reference herein.

Supercritical fluids may be used as an alternative to solvents for small molecule extraction. In more detail, supercritical fluids may be used to extract small molecules such as fats, lipids and other cellular debris from the animal-derived tissue. Supercritical fluids may be exposed to the tissue for a set time and temperature, processing the animal-derived tissue. This eliminates the need to post-process (e.g., washing, drying, etc.) and may result in a purified material.

In other embodiments, supercritical fluids are used as a solvent to carry a solute (e.g., a cross-linking agent) to cross-link animal-derived tissue. Alternatively, supercritical fluids may be used as an alternative to traditional cross-linking agents such as isocyanates or aldehydes.

In embodiments, at least a portion of the animal-derived tissue may contact the supercritical fluid. The tissue may be placed in a reactor and the reactor may be infused with the supercritical fluid. The tissue may then be transferred to another reactor to carry out any additional required steps for tissue processing. Alternatively, tissue may be processed with supercritical fluids, followed by the normal tissue processing (with solvents) and then cross-linking with cross-linking agents and/or supercritical fluids sequentially in the same reactor.

The animal-derived tissue may be washed to remove any residual chemicals or cellular debris. Washing may be carried out at a temperature from about 30 °C to about 50 °C. To ensure that all of the residual chemicals and/or cellular debris have been eliminated, a sweep is advantageously carried out with an inert gas at low pressure.

Lastly, sterilization of the animal-derived tissue may be carried out, utilizing methods including those within the purview of those skilled in the art.

The resulting processed and cross-linked animal-derived tissue may then be used to form various medical devices suitable for a variety of surgical and wound applications. The medical devices according to the present disclosure may be any structure suitable for being attached or implanted into tissue, body organs or lumens, including, but not limited to, films, foams, slit sheets, pledgets, tissue grafts, stents, scaffolds, buttresses, wound dressings, meshes, and/or tissue reinforcements.

The resulting devices may be used, for example, for closing and healing visceral wall defects and incisions, including incisions due to the removal of tumors, wounds, anastomoses, and fistulae. The medical devices can improve the healing of a gastro-intestinal anastomosis and may provide an effective approach to the management and prevention of the formation of fistula. The medical devices may also prevent complications of polypectomy (e.g., bleeding and perforation).

The medical devices may further be used for delivery of a bioactive agent. Thus, in some embodiments, at least one bioactive agent may be provided in or on the animal-derived tissue. In embodiments, the bioactive agent may be dissolved in the supercritical composition to allow for incorporation of the bioactive agent into a polymer and/or the animal-derived tissue. The bioactive agent(s) can then penetrate the substrate material with the aid of the supercritical fluid, as described in U.S. Patent Publication No. 2009/0269480, the entire disclosure of which is incorporated by reference herein.

The term "bioactive agent," as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent that provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present medical device in any suitable form of matter, e.g., films, powders, liquids, gels and the like.

Examples of classes of bioactive agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Anti-adhesive agents can be used to prevent adhesions from forming between the implantable medical device and the surrounding tissues opposite the target tissue. In addition, anti-adhesive agents may be used to prevent adhesions from forming between the coated implantable medical device and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the present disclosure.

Other bioactive agents include local anesthetics, non-steroidal antifertility agents, parasympathomimetic agents, psychotherapeutic agents, tranquilizers, decongestants, sedative hypnotics, steroids, sulfonamides, sympathomimetic agents, vaccines, vitamins, antimalarials, anti-migraine agents, anti-parkinson agents such as L-dopa, anti-spasmodics, anticholinergic agents (e.g., oxybutynin), antitussives, bronchodilators, cardiovascular agents such as coronary vasodilators and nitroglycerin, alkaloids, analgesics, narcotics such as codeine, dihydrocodeinone, meperidine, morphine and the like, non-narcotics such as salicylates, aspirin, acetaminophen, d-propoxyphene and the like, opioid receptor antagonists, such as naltrexone and naloxone, anti-cancer agents, anti-convulsants, anti-emetics, antihistamines, anti-inflammatory agents such as hormonal agents, hydrocortisone, prednisolone, prednisone, non-hormonal agents, allopurinol, indomethacin, phenylbutazone and the like; prostaglandins and cytotoxic drugs, chemotherapeutics, estrogens, antibacterials, antibiotics, anti-fungals, anti-virals, anticoagulants, anticonvulsants, antidepressants, antihistamines, and immunological agents.

Other examples of suitable bioactive agents also include viruses and cells, peptides, polypeptides and proteins, analogs, muteins, and active fragments thereof, such as immunoglobulins, antibodies, cytokines (e.g., lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone), vaccines (e.g., tumoral, bacterial and viral antigens), somatostatin, antigens, blood coagulation factors, growth factors (e.g., nerve growth factor, insulin-like growth factor), bone morphogenic proteins, TGF-B, protein inhibitors, protein antagonists, and protein agonists, nucleic acids, such as antisense molecules, DNA, RNA, RNAi, oligonucleotides, polynucleotides, and ribozymes.

It will be appreciated that the above-disclosed and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, steps or components of claims should not be implied or imported from the specification or any other claims as to any particular order, number, position, size, shape, angle, or material.

The invention may be described by reference to the following numbered paragraphs:-
1. A method comprising:
   contacting at least one animal-derived tissue with a supercritical fluid;
   processing the animal-derived tissue with the supercritical fluid; and,
   recovering the processed animal-derived tissue.
2. The method according to paragraph 1, wherein the supercritical fluid is selected from the group consisting of supercritical nitrogen dioxide, supercritical carbon dioxide, and combinations thereof.
3. The method according to paragraph 1, wherein the animal-derived tissue is selected from the group consisting of consisting of proteins, peptides, polysaccharides and combinations thereof.
4. The method according to paragraph 1, wherein the animal-derived tissue comprises collagen.
5. The method according to paragraph 1, wherein processing the animal-derived tissue comprises the step of extracting small molecules from the animal-derived tissue.
6. A method comprising:
   dissolving a cross-linking agent in a supercritical fluid;
   contacting at least one animal-derived tissue with the supercritical fluid;
   cross-linking the animal-derived tissue the cross-linking agent;and,
   recovering the cross-linked animal-derived tissue.
7. The method according to paragraph 6, wherein the supercritical fluid is selected from the group consisting of supercritical nitrogen dioxide, supercritical carbon dioxide, and combinations thereof.
8. The method according to paragraph 6, wherein the animal-derived tissue is selected from the group consisting of consisting of proteins, peptides, polysaccharides, and combinations thereof.

## Claims

1. A method comprising:
contacting at least one animal-derived tissue with a supercritical fluid;
processing the animal-derived tissue with the supercritical fluid; and,
recovering the processed animal-derived tissue.

2. The method according to claim 1, wherein the supercritical fluid is selected from the group consisting of supercritical nitrogen dioxide, supercritical carbon dioxide, and combinations thereof.

3. The method according to claim 1, wherein the animal-derived tissue is selected from the group consisting of consisting of proteins, peptides, polysaccharides and combinations thereof.

4. The method according to claim 1, wherein the animal-derived tissue comprises collagen.

5. The method according to claim 1, wherein processing the animal-derived tissue comprises the step of extracting small molecules from the animal-derived tissue.

6. A method comprising:
dissolving a cross-linking agent in a supercritical fluid;
contacting at least one animal-derived tissue with the supercritical fluid;
cross-linking the animal-derived tissue the cross-linking agent;and,
recovering the cross-linked animal-derived tissue.

7. The method according to claim 6, wherein the supercritical fluid is selected from the group consisting of supercritical nitrogen dioxide, supercritical carbon dioxide, and combinations thereof.

8. The method according to claim 6, wherein the animal-derived tissue is selected from the group consisting of consisting of proteins, peptides, polysaccharides, and combinations thereof.
